# EUROPEAN PATENT APPLICATION

(11) **EP 2 371 274 A1**
(43) Date of publication of application: **05.10.2011**
(21) Application number: 09836104.1
(22) Date of filing: 03.12.2009
(51) Int. Cl.: A61B 5/00

(54) **PNEUMATIC SELF-SYNCHRONIZING SOMATOSENSORY STIMULATION SYSTEM AND METHOD**

(30) Priority: 30.12.2008 ES 200803750
(71) Applicant: Universidad Politécnica de Madrid, 28040 Madrid (ES)
(72) Inventor: MAESTÚ UNTURBE, Ceferino, E-28040 Madrid (ES); CORTÉS DE CASTRO, Álvaro, E-28040 Madrid (ES); VÁZQUEZ MÉNDEZ, José Manuel, E-28040 Madrid (ES); TENEMBAUN, Emanuelle, E-28040 Madrid (ES); DEL POZO GUERRERO, Francisco, E-28040 Madrid (ES)
(74) Representative: Carvajal y Urquijo, Isabel
(86) International application number: PCT/ES2009/000558
(87) International publication number: WO 2010/076351

(57) **Abstract**

**SUMMARY**

Pneumatic self-synchronizing somatosensory stimulation system with magnetoencephalography and/or magnetic resonance devices having a trigger comprising:
- A first management subsystem which comprises a portable computer (7);
- A second control subsystem which comprises a data acquisition board (9) which synchronize automatically the signals generated by the computer (7) and the information received from the trigger (10) of the measurement device, communicating with the system of valves of the mechanical subsystem by means of at least one connection cable (11);
- A third mechanical subsystem which comprises a system of at least two valves: a proportional valve (14) and a non-proportional valve (15), which are connected to the application subsystem;
- A fourth application subsystem which comprises an application support (2) with at least two connections (4, 5) to the valves of the mechanical module which regulate the extension and retraction movement of a piston (1).

## Description

### Field of the invention

The present invention refers to a pneumatic synchronized somatosensory stimulation compatible with magnetoencephalography (MEG) and magnetic resonance (MG), which is included in the field of Biomedical Engineering, specifically to studies developed in a work environment with special conditions, such as those in a shielded room with the electromagnetic field (EMF) of the brain activity record system MEG (magnetoencephalography).

### Background of the invention

Most existing tests, studies and methods for the characterization of pain use a type of stimulation produced with electric current applied on the skin (P: 6146334, P: 6113552), or with heat applied in any part of the body (P: 5941833) and all of them are based on the subjective interpretation of the individual response to the pain (P: 6807965), without resorting to objective indicators, such as the brain processing of the signal. Thus, there is a need to objectively characterize the pain through analysis techniques, such as the new brain activity record technique based on the capture of weak brain electromagnetic fields product of the responses obtained after stimulation activity, such as the magnetoencephalography. To that end, there is a need to develop devices capable of generating a controlled intensity stimulation known at all times, capable of interacting, synchronizing the capture system with the stimulation procedure of the MEG device.

The revision of different articles and related patents with the somatosensory stimulation leads us to obtaining the following results of the state of the art:

Most verified registered patents, related to the stimulation performed within a magnetoencephalography device, base their application on the direct stimulation of brain tissue. To that end, they place a small generator in the subject's chest and through cable and electrode directly stimulate the brain through electrical pulse trains, having to perform the recording outside the shielded room and not in a synchronized manner, besides it is a highly invasive procedure (US Pat. 11078114, US Pat. 10583630).

As regards the somatosensory stimulation, we also find devices which simulate environments generating auditory and visual somatosensory signals and which are used, for example, for the vestibular rehabilitation and neuronal disorders. Like these, we also find more detailed studies where the brain activity is recorded (Doctoral dissertation: Effects of the thermoanalgesic studies on subcortical reflex circuits, Misericordia Veciana de las Heras), but in this case the stimulation is performed in a thermal manner, by means of a laser device through heat. Another stimulation system is the so-called Dodecapus (Ruey-Song Huang, and Martin I. Sereno. Dodecapus: An MR-compatible system for somatosensory stimulation), which is also based on a software-controlled pneumatic system, but in this case air is directly applied on the subjects face, but due to its construction its application is not compatible with MEG systems.

### Description of the invention

The present invention refers to somatosensory stimulators compatible and automatically synchronized with the operating conditions of magnetoencephalography (MEG) devices, and which may also be compatible with magnetic resonance devices having a trigger.

This stimulator has a general modular structure divided in four subsystems: A) Management, B) Control, C) Mechanical, and D) Application.

### A) Management Subsystem

### Formed by a portable computer as the user interface.

### B) Control Subsystem

It is formed by a programmable data acquisition board connected to the portable computer of the management system for the automatic synchronization with the magnetoencephalography (MEG) device.

The board has at least one synchronization input which is connected with the magnetoencephalography (MEG) so that it provides a software exact synchronization; that is, the trigger system of the MEG produces a series of signals during its operation which are those which make it work in a perfectly synchronized manner. Also, it can be synchronized with magnetic resonance devices as long as they have a trigger.

To prevent the connections between the board and cables from being exposed, all the subsystem is protected through an exterior cover.

The board contains analog outputs which can be voltage or current ones, and which are those which generate, the signals to control the valves of the mechanical subsystem.

### C) Mechanical Subsystem

It comprises two parts:
- an exterior one formed by an air compressor connected to a regulation filter, and
- another one built-in in a metallic box with the same characteristics.

The air compressor is connected to the regulation filter by means of a plastic tube. The outlet of the regulation filter is connected to a system of two valves located inside the metallic box, a proportional valve and another non-proportional valve. The proportional valve is controlled as regards voltage to reduce the load of the power supply and render the general system more portable. In this way, the general pneumatic system is made of methacrylate with reduced dimensions compared to what is previously known.

On the other hand, the compressed air system used generates a maximum pressure of 10bars, which enables to adjust the pressure level applied in the experiences which are performed in the magnetoencephalography, avoiding any intensity fluctuation. This adjustment is due to the fact that the measures taken in the magnetoencephalography are more critical when much smaller magnetic field intensities are used.

The stimulating device allows the stimulation in five possible modes.

It is not an object of this document to deal with the software content, but the versatility of the different modes is a fundamental part of the invention, and they are presented hereinbelow as part of the description:

A mode called "normal", which allows a fixed sequence determined by the user. It enables to select amplitude between 0 and 100, proportional to the intensity of the pressure to be provided, and it enables to select the duration of the pulse applied and the interval between the successive applications. Next, it generates a pulse train witch these data.

A mode to limit the threshold of pain of the subject. It enables to select a jump, from which a controlled growing sequence of 0 to 100 is generated, proportional to the pressure intensity to be provided, following the given jump, in which the duration of the pulses and the interval between them can be selected.

Two sequence random reproduction modes in which the stimulation parameters vary freely. One of them enables to select the maximum and minimum margins between which the amplitude values will range and the other varies the values randomly between 0 and 100.

A personalized mode to characterize each one of the individual parameters. It enables to individually select the amplitude of each pulse, as well as the duration of the pulses and the interval between them. Next, a pulse train is made with the data inserted.

After the activation, each one of the modes shows in a graphic manner a representation of the sequence to be applied.

### D) Application Subsystem

The outputs of the two aforementioned valves are connected with the application system through plastic tubes with the same characteristics as the aforementioned in the description of the mechanical subsystem, but with a maximum length of 7 meters, which enables to prevent considerable pressure losses on the tube walls. These two tubes are connected to an anatomic applicator made of a cushioned plastic material. This applicator has fastenings which are Velcro® type adhesive strips which secure a firm and accurate fixing in the desired point.

The stimulation is performed through a plastic piston firmly joined to a plate which is also made of plastic, to prevent its displacement, which makes an extension-retraction movement. For the extension movement, the action of the proportional valve is used. This, through software, generates a pressure which results in a proportional extension of the piston. For the retraction movement, the action of the non-proportional valve is used.

The application system is designed to be adjustable to different parts of the human body. To that end, the piston system is mounted on an adaptable semi-rigid surface made of non-ferromagnetic material, where there are placed a series of strips which are also made of non-ferromagnetic material, which serve as anchoring devices to the human body. The applicator also has measures to prevent the swelling of the application points, as well as possible injuries.

The pneumatic somatosensory stimulation procedure which uses the device of the invention for measuring through magnetoencephalography or magnetic resonance the response generated in the brain, and thus obtaining a characterization of the pain, can be summarized in the following stages:
- A first stage of selection of the stimulation mode in the computer of the management subsystem, the system being ready for its activation through the trigger signal.
- A second automatic synchronization stage in the data acquisition board of the control subsystem, of the stimulation produced by the piston, with the signal detected in the board being received through the trigger of the magnetoencephalography.
- A third stage of conversion of the signals generated by the computer in the data acquisition board of the control subsystem.
- A fourth stage of sending the signal of the data acquisition board to the valves of the mechanical subsystem, so that the selected pressure is generated.
- A fifth stage of sending the pressure of the valves to the connections of the application subsystem.
- A sixth stage of extension and retraction of the piston of the application subsystem.
- A seventh stage of measurement, in the magnetoencephalography or magnetic resonance devices, of the signal produced by the brain in response to the stimulation produced.

### Brief description of the drawings

The following is a brief description of a series of drawings which will help understand the invention better relating to an embodiment of said invention which is presented as a non-limiting example thereof.
Figure 1 shows a perspective view of the application subsystem.
Figure 2 shows the rear connection of the applicator, with the two inputs for the plastic tubes coming from the proportional valve and the non-proportional valve.
Figure 3 shows a scheme of the connection through USB port between the management subsystem and the control subsystem which is synchronized with the MEG trigger.
Figure 4 shows a scheme of the assembly between the board and the proportional and non-proportional valves, and how these are connected to the applicator.

The elements represented in the figures correspond to the following numeric references:
1.- Piston
2.- Applicator
3.- Cushioning
4.- Connection to the proportional valve
5.- Connection to the non-proportional valve
6.- Strips
7.- Computer
8.- USB port
9.- Data acquisition board
10.- Trigger
11.- Connection cables with the valve system
12.- Analogic outputs of the data acquisition board
13.- Digital connections of the data acquisition board
14.- Proportional valve
15.- Non-proportional valve

### Description of a preferred embodiment of the invention

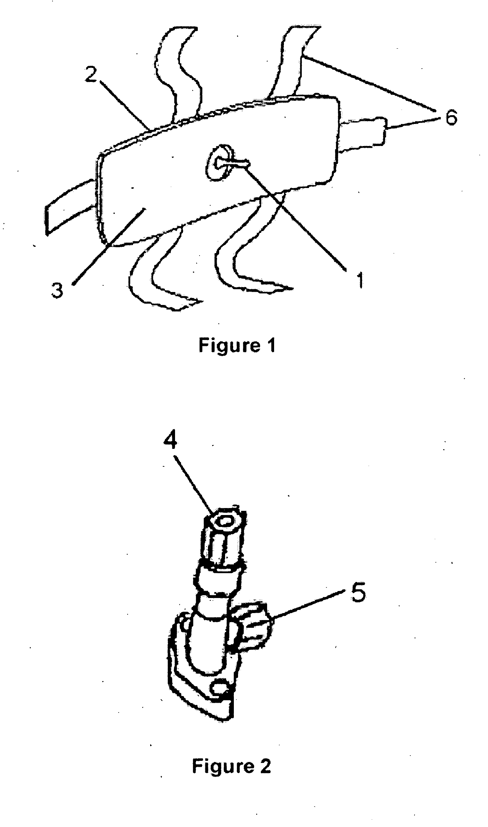

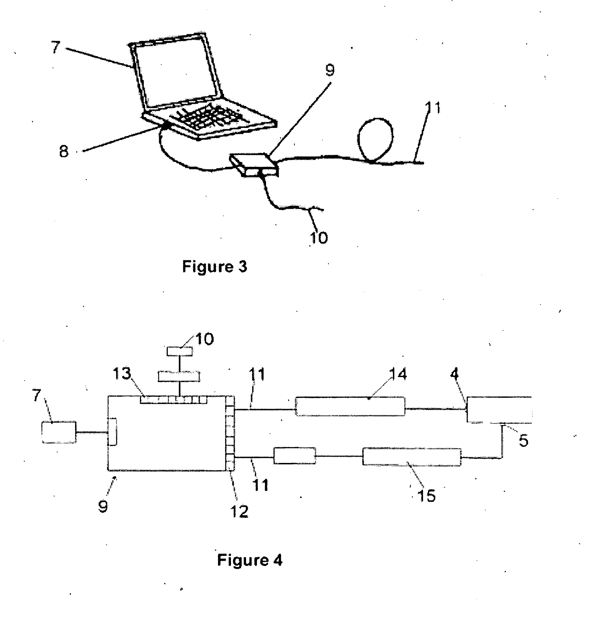

In view of the aforementioned, the present invention refers to a pneumatic somatosensory stimulator compatible with magnetoencephalography (MEG) devices, which incorporates an application subsystem with a plastic piston (1) which is extended and retracted by pneumatic pressure within an applicator which can be adapted to different body parts (2), and covered by a cushioning (3), as it can be seen in figure 1 It has two connections to the valve system, a connection (4) with the proportional valve (14) and another connection (5) with the non-proportional valve (15), as it can be seen in figures 2 and 4, and it also has a series of Velcro® type adhesive strips (6) which allow its strong fastening to the body. This pneumatic pressure which moves the piston is supplied by an a compressed air pump which communicates through a plastic cable with a regulation filter, and from this to the valve system, and which form the mechanical subsystem of the system.

The management system has a portable computer (7) which supports the control software and user interface. This computer is connected by means of one of its USB ports (8) to a data board (9) which constitutes the control subsystem and which performs the synchronization with the trigger (10) of the MEG. In turn, the board communicates with the valve system through a cable which has a maximum 25-meter length (11). The data acquisition board, like the DT9853-M board, as data processing means, is the one in charge of converting the signals generated by the computer in the control program, involved in the operation of the stimulation system. The board has 8 analog outputs (12) which can be voltage or current ones, and which are the ones that generate the signals to control the proportional valve. In this case, we use them as voltage ones. Also, it has 8 digital inputs and 8 outputs (13). One of the digital outputs is used to control the non-proportional valve. One of the digital inputs is the one used for the synchronization with the trigger of the magnetoencephalography. A graphic interface supported on Matlab language can be implemented, which is the one used to control the DT9853-M board.

It is understood that, without altering the essence of the invention, changes can be made regarding materials, shape and size and the arrangement of the elements within the characterization thereof.

The terms used in the description and the sense thereof must be considered in a non-limiting manner.

## Claims

1. Pneumatic self-synchronizing somatosensory stimulation system **characterized in that** it comprises four systems:
- A first management subsystem which comprises a portable computer (7);
- A second control subsystem which comprises a data acquisition board (9) which synchronizes automatically the signals generated by the computer (7) and the information received from the trigger (10) of the measurement device, communicating with the system of valves of the mechanical subsystem by means of at least one connection cable (11);
- A third mechanical subsystem which comprises a system of at least two valves: a proportional valve (14) and a non-proportional valve (15), which are connected to the application subsystem;
- A fourth application subsystem which comprises an application support (2) with at least two connections (4, 5) to the valves of the mechanical module which regulate the extension and retraction movement of a piston (1);
all of which configured to be compatible with magnetoencephalography and/or magnetic resonance measurement devices having a trigger.

2. Pneumatic stimulation system according to claim 1 **characterized in that** the computer of the management subsystem is a portable computer (7) which houses the control software of the data acquisition board (9) to which it is connected.

3. Pneumatic stimulation system according to claims 1-2 **characterized in that** the control and management subsystems are connected through a USB connection.

4. Pneumatic stimulation system according to the preceding claims **characterized in that** the data acquisition board (9) of the control subsystem has at least two analog outputs which can be voltage or current outputs, and which are the ones that generate the signals to control the valves, both proportional (4) and non-proportional (15).

5. Pneumatic stimulation system according to claim 4 **characterized in that** the proportional valve is voltage controlled, which makes the general system more portable.

6. Pneumatic stimulation system according to the preceding claims **characterized in that** the data acquisition board has at least one input and at least one digital output, one of which is the one used for the synchronization with the trigger of the magnetoencephalography.

7. Pneumatic stimulation system according to claims 4-6 **characterized in that** the data acquisition board has 8 analog connections and 8 inputs and 8 digital outputs.

8. Pneumatic stimulation system according to the preceding claims **characterized in that** the data acquisition board (9) of the control subsystem communicates with the proportional and non-proportional valves (14, 15) of the mechanical subsystem through a cable (11) which has a maximum length of 25 meters.

9. Pneumatic stimulation system according to the preceding claims **characterized in that** the mechanical subsystem comprises an air compressor connected to a regulation filter through a tube.

10. Pneumatic stimulation system according to claim 7 **characterized in that** the final maximum pressure to the filter output is 10 bars.

11. Pneumatic stimulation system according to the preceding claims **characterized in that** the application subsystem comprises an applicator (2) made of a non-ferromagnetic semi-rigid polymer adjustable to any part of the human body.

12. Pneumatic stimulation system according to the preceding claims **characterized in that** the application subsystem comprises some adjustable strips (6) made of a non-ferromagnetic material.

13. Pneumatic somatosensory stimulation method **characterized in that** the pneumatic system according to claims 1-12 is **characterized in that** it comprises:
- A first stage of selection of the stimulation mode in the computer (7) of the management subsystem, the system being ready for its activation through the trigger signal.
- A second automatic synchronization stage in the data acquisition board (9) of the control subsystem, of the stimulation produced by the piston, with the signal detected in the board being received through the trigger of the magnetoencephalography.
- A third stage of conversion of the signals generated by the computer (7) in the data acquisition board (9) of the control subsystem.
- A fourth stage of sending the signal of the data acquisition board (9) to the valves (14, 15) of the mechanical subsystem, so that the selected pressure is generated.
- A fifth stage of sending the pressure of the valves to the connections of the application subsystem (4, 5).
- A sixth stage of extension and retraction of the piston (1) of the application subsystem.
- A seventh stage of measurement, in the magnetoencephalography or magnetic resonance devices, of the signal produced by the brain in response to the stimulation produced.

14. Pneumatic somatosensory stimulation method according to claim 13 **characterized in that** in the first stage a mode is selected between the following different stimulation modes: A mode of establishing one fixed sequence, another mode of limiting a threshold, two modes of random reproduction of sequences, and a personalized mode to characterize each one of the individual parameters.
